# EUROPEAN PATENT SPECIFICATION

(11) **EP 1 724 343 B1**
(45) Date of publication and mention of the grant of the patent: **07.04.2010**
(21) Application number: 05710653.6
(22) Date of filing: 24.02.2005
(51) Int. Cl.: C12N 15/09, C07H 21/04, C12N 9/22

(54) **DNA ENZYME AND METHOD OF CONTROLLING THE ACTIVITY THEREOF**
DNA-ENZYM UND VERFAHREN ZUR STEUERUNG DESSEN AKTIVITÄT
ENZYME D'ADN ET PROCEDE DE CONTROLE DE L'ACTIVITE DE CELLE-CI

(30) Priority: 27.02.2004 JP 2004055086
(43) Date of publication of application: 22.11.2006
(73) Proprietor: Japan Science and Technology Agency, Kawaguchi-shi, Saitama 332-0012 (JP)
(72) Inventor: ASANUMA, Hiroyuki, Kawaguchi-shi, Saitama 332-0021 (JP); KOMIYAMA, Makoto, Tokyo 150-0022 (JP); MATSUNAGA, Daijiro, Tokyo 133-0054 (JP); KURAMOCHI, Takeshi, Nerima-ku, Tokyo 177-0034 (JP)
(74) Representative: Jappy, John William Graham
(86) International application number: PCT/JP2005/003052
(87) International publication number: WO 2005/083073

(56) References cited:
- MEI SHIRLEY H J ET AL: "An efficient RNA-cleaving DNA enzyme that synchronizes catalysis with fluorescence signaling" JOURNAL OF THE AMERICAN CHEMICAL SOCIETY, AMERICAN CHEMICAL SOCIETY, WASHINGTON, DC, US, vol. 125, no. 2, 15 January 2003 (2003-01-15), pages 412-420, XP002245985 ISSN: 0002-7863
- YAMAZAWA T. ET AL: 'Kakusan Kino no Hikari Seigyo o Mezashita Shushoku Oligonucleotide no Sekkei (Photo-regulation of RNA cleavage by DNA enzyme carrying azobenzene)' POLYMER PREPRINTS, JAPAN. vol. 50, no. 5, 2001, page 977, XP002997263
- SANTORO S.W. ET AL: 'A general purpose RNA-cleavings DNA enzyme.' PROC.NATL.ACAD.SCI.USA. vol. 94, no. 9, 1997, pages 4262 - 4266, XP001009844
- ASANUMA H. ET AL: 'Photo-responsive ologonucleotides carrying azobenzene in the side-chains.' TETRAHEDRON LETTERS. vol. 39, no. 49, 1998, pages 9015 - 9018, XP002934800
- LIU Y. ET AL: 'Light-regulated Catalysis by an RNA-cleaving Deoxyribozyme.' JOURNAL OF MOLECULAR BIOLOGY. vol. 341, no. 4, August 2004, pages 887 - 892, XP004743264
- KURAMOCHI T. ET AL: 'Azobenzene Donyu ni yoru DNA Enzyme no Kokinota (Functionalization of DNA enzyme by introduction of azobenzine residues)' CSJ: THE CHEMICAL SOCIETY OF JAPAN KOEN YOKOSHU. vol. 84, no. 2, 11 March 2004, page 1070, XP002997264

## Description

### Technical Field

The present invention relates to a DNA enzyme and a method for controlling the activity thereof. In particular, it relates to a DNA enzyme having the RNA cleavage activity significantly improved as compared with that of a DNA enzyme simply composed of four natural bases and a method for controlling the activity of a DNA enzyme by light irradiation at specific wavelengths.

### Background Art

If an RNA can be selectively hydrolyzed on a sequence basis, gene expression at a messenger RNA level can be suppressed, and an application to the therapy for diseases based on genes can be expected. Naturally occurring RNase is not a protein. It is simply composed of an RNA, and is referred to as ribozyme. However, the RNA is unstable and tends to be decomposed. Therefore, a more stable DNA hydrolytic enzyme (artificial enzyme) has been required. In response to the requirement, Santoro et al., in the U.S., have proposed an RNA hydrolytic enzyme simply composed of a naturally occurring DNA in 1997 for the first time in the world (Santoro et al, (1997) Proc. Natl. Acad USA 94, 4262 - 4266).

The RNA hydrolytic enzyme simply composed of a DNA is generally referred to as a DNA enzyme (deoxyribozyme, DNAzyme), and is an artificial ribonuclease developed by an in vitro selection method. Since an in vivo metal, Mg²⁺, serves as a cofactor, an in vivo application is possible. Specific contents thereof are disclosed by Santoro et al. Supra. An 8-17 DNA enzyme and 10-23 DNA enzyme are included, and sequence formulae thereof are as described below.

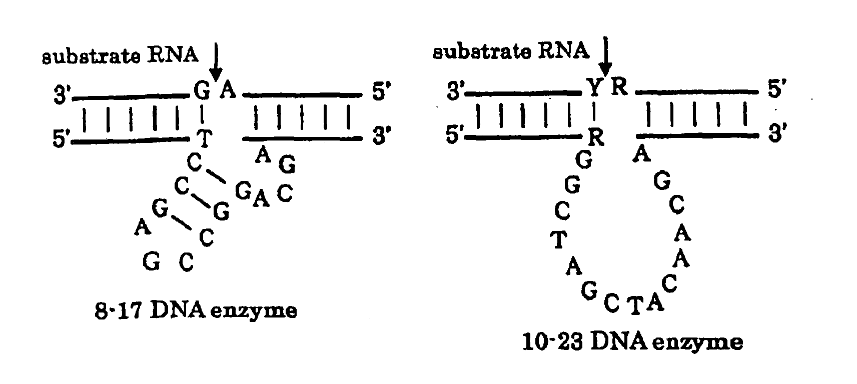

In the above-described sequence formulae, arrows indicate cleavage sites. The base sequence of the substrate RNA at the cleavage site is GA for the 8-17 DNA enzyme, and is Y(U or C)R(A or G) for the 10-23 DNA enzyme. The sequence of the DNA enzyme becomes a sequence complementary to the substrate RNA. However, CCGAGCCGGACGA (sequence number 1) in the 8-17 DNA enzyme and GGCTAGCTACAACGA (sequence number 2) in the 10-23 DNA enzyme are catalytically active loops, and are not complementary to the substrate RNA.

On the other hand, Hiroyuki Asanuma (2003) J.Japanese Society for Biomaterials 21, 290-296 reports the gene expression control by the light irradiation, and the gene expression control is conducted by using an artificial DNA in which azobenzene has been introduced in a side chain of the DNA. Specifically, since reversible structural isomerization between a trans form (planar structure) and a cis form (nonplanar structure) of azobenzene is effected by light irradiation at specific wavelengths, it becomes possible to, for example, optically control the formation and dissociation of a duplex of the DNA and optically control the formation of a triplex by taking advantage of this characteristic of azobenzene.

### Disclosure of Invention

For the DNA enzyme disclosed by Santoro et al Supra, the RNA cleavage activity itself is not high, and is very low as compared with that of natural ribozyme. Consequently, the DNA enzyme has been required to have higher activity.

It is believed that the control of the RNA cleavage activity of the DNA enzyme is very difficult. Therefore, if the activity can be reversibly controlled by an external stimulus, e.g., light irradiation, without changing the condition in the reaction system, the usefulness thereof can be increased significantly.

Accordingly, it is an object of the present invention to provide a DNA enzyme having the RNA cleavage activity significantly improved as compared with those of known DNA enzymes.

It is another objective of the present invention to provide a method for controlling the activity, the method being capable of reversibly controlling the RNA cleavage activity of the DNA enzyme by light irradiation.

### Means for Solving the Problems

In order to overcome the above-described problems, the inventors of the present invention have conducted intensive research. As a result, it has been found that the above-described objective can be achieved by introducing a nucleotide residue having a planar structure at a predetermined site of a DNA enzyme.

That is, the DNA enzyme is characterized by including a nucleotide residue, to which an azobenzene group, or a derivative thereof, is bonded at a 3'-side end of a catalytically active loop of the DNA enzyme.

The inventors of the present invention have found that reversible structural isomerization from the above-described planar structure to a nonplanar structure has been able to be effected by applying the light at a specific wavelength to the above-described DNA enzyme, and the RNA cleavage activity of the DNA enzyme is then able to be controlled.

The method for controlling the activity according to an aspect of the present invention is characterized by including the step of applying light at specific wavelengths to the DNA enzyme including a nucleotide residue, to which an azobenzene group, or a derivative thereof, is bonded and thereby effecting reversible structural isomerization between a planar structure and a nonplanar structure of the organic group, so as to control the RNA cleavage activity of the DNA enzyme.

### Advantages

For the DNA enzyme including a nucleotide residue having a planar structure of the present invention, the RNA cleavage activity is significantly improved as compared with that of a DNA enzyme simply composed of four natural bases. According to the method for controlling the activity of a DNA enzyme of the present invention, it becomes possible to reversibly control the cleavage activity of the DNA enzyme by light irradiation at specific wavelengths, and it can be expected that in vivo gene expression is optically controlled.

### Best Mode for Carrying Out the Invention

The preferable embodiments of the present invention will be specifically described below.

The DNA enzyme according to the present invention is a chemically modified DNA enzyme, wherein a nucleotide residue, to which an azobenzene group, or a derivative thereof, is bonded has been introduced at a 3'-side end of a catalytically active loop of the DNA enzyme disclosed by Santoro et al. Supra. The base sequence of the above-described DNA enzyme, except the catalytically active loop, is a base complementary to the substrate RNA. However, the base sequence of the RNA enzyme is not specifically limited.

The DNA enzyme according to the present invention is represented by the following Formulae. 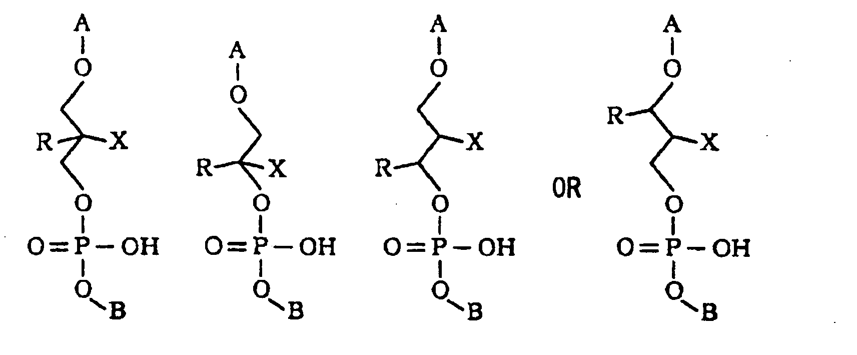 In the above-described Formulae, A represents a catalytically active loop end, B represents nucleotide or oligonucleotide. X represents an azobenzene group, or a derivative thereof. R represents an unsubstituted or a halogen atom-, hydroxyl-, amino-, nitro-, or carboxyl-substituted alkyl group or alkoxy group having the carbon number of 1 to 20, preferably of 1 to 10, and more preferably of 1 to 4; an unsubstituted or a halogen atom-, hydroxyl-, amino-, nitro-, or carboxyl-substituted alkenyl group or alkynyl group having the carbon number of 2 to 20, preferably of 2 to 10, and more preferably of 2 to 4; a hydroxyl group; a halogen atom; an amino group; a nitro group; or a carboxyl group.

Any group may be intercalated in a portion bonded to the nucleotide residue. Examples of X can include organic groups represented by the following Formula (I), (II), or (III).

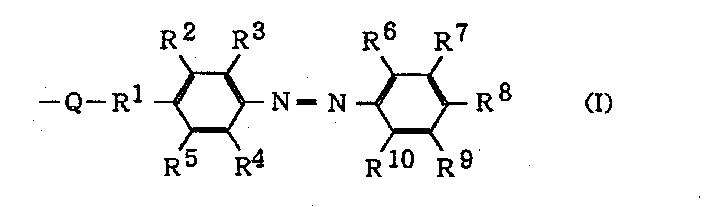

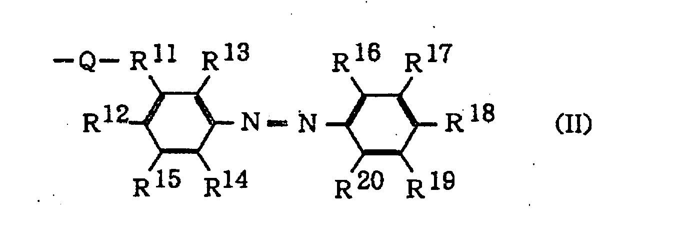

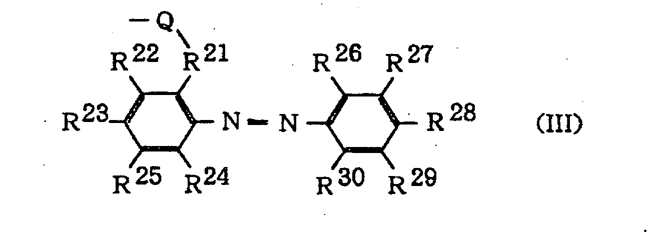

In the above-described Formulae (I), (II), and (III), R¹, R¹¹, and R²¹ represent independently a direct bond; an unsubstituted or a halogen atom-, hydroxyl-, amino-, nitro-, or carboxyl-substituted alkylene group having the carbon number of 1 to 20, preferably of 1 to 10, and further preferably of 1 to 4; or an unsubstituted or a halogen atom-, hydroxyl-, amino-, nitro-, or carboxyl-substituted alkenylene group having the carbon number of 2 to 20, preferably of 2 to 10, and further preferably of 2 to 4. Q represents a direct bond, an oxygen atom, a -(CH₂)ₙ-NH-CO- group, or a -(CH₂)ₙ-CO-NH- group, where n = 1 to 5. R² to R¹⁰, R¹² to R²⁰, and R²² to R³⁰ represent independently an unsubstituted or a halogen atom-, hydroxyl-, amino-, nitro-, or carboxyl-substituted alkyl group or alkoxy group having the carbon number of 1 to 20, preferably of 1 to 10, and further preferably of 1 to 4; an unsubstituted or a halogen atom-, hydroxyl-, amino-, nitro-, or carboxyl-substituted alkenyl group or alkynyl group having the carbon number of 2 to 20, preferably of 2 to 10, and further preferably of 2 to 4; a hydroxyl group; a halogen atom; an amino group; a nitro group; or a carboxyl group.

The synthesis of the DNA enzyme including the nucleotide residue, according to the present invention, can be conducted in accordance with known techniques, e.g., techniques described in The Journal of Organic Chemistry 62. 846-852 (1997), Tetrahedron Letters 39. 9019-9022 (1998), and Angewandte Chemie International edition 40. 2671-2673 (2001). Phosphoamidite monomers corresponding to individual nucleotide residues are synthesized, a known DNA synthesizer is used and, thereby, DNA enzymes including desired nucleotide residues can be synthesized. In this case, polymethylene chains having various lengths can be used. However, an unsubstituted or an alkyl-substituted ethylene chain or a trimethylene chain is preferable. In this case, preferably, the azobenzene group is introduced such that it forms a covalent bond with any one of the carbon atoms of the ethylene chain, or with a central carbon atom of the trimethylene chain.

A method for controlling the RNA cleavage activity of the DNA enzyme will be described below. A DNA enzyme including a nucleotide residue, to which an azobenzene group, or a derivative thereof, is bonded is used and is irradiated with light at specific wavelengths, wherein structural isomerization between a planar structure and a nonplanar structure of the organic group is effected by the light irradiation at specific wavelengths. Consequently, reversible structural isomerization between a planar structure and a nonplanar structure of the above-described azobenzene group is effected and it becomes possible to control the RNA cleavage activity. Here, the base sequence of the DNA enzyme except the catalytically active loop is a base complementary to the substrate RNA. However, the base sequence of the RNA is not specifically limited.

When the introduction position of the above-described nucleotide residue is the 3'-side end of the catalytically active loop, the DNA enzyme according to the present invention is derived, and a high RNA cleavage activity is exhibited. However, in the method for controlling the activity according to the present invention, the above-described introduction position is not specifically limited, and may be an oligonucleotide complementary to the substrate RNA. Such a DNA enzyme is represented by the following Formulae.

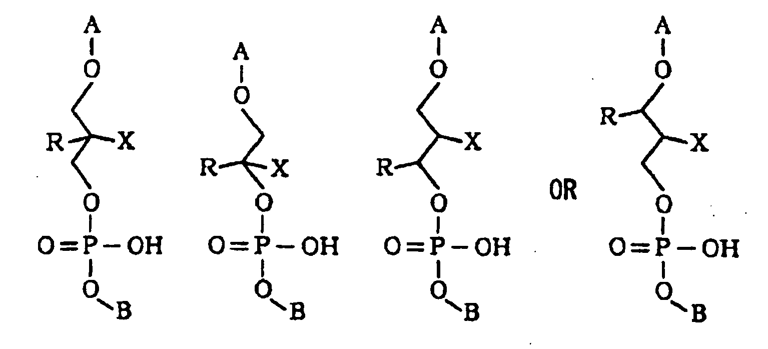

In the above-described Formulae, A and B represent independently a hydrogen atom, nucleotide or oligonucleotide. However, A and B do not represent a hydrogen atom at the same time. X represents an azobenzene group, or a derivative thereof. R represents an unsubstituted or a halogen atom-, hydroxyl-, amino-, nitro-, or carboxyl-substituted alkyl group or alkoxy group having the carbon number of 1 to 20, preferably of 1 to 10, and more preferably of 1 to 4; an unsubstituted or a halogen atom-, hydroxyl-, amino-, nitro-, or carboxyl-substituted alkenyl group or alkynyl group having the carbon number of 2 to 20, preferably of 2 to 10, and more preferably of 2 to 4; a hydroxyl group; a halogen atom; an amino group; a nitro group; or a carboxyl group.

The benzene ring may have any substituent as long as the function of controlling the enzyme activity through reversible structural isomerization by the light irradiation is not impaired, and any group may be intercalated in a portion bonded to the nucleotide residue. Preferably, a substituent and an intercalating group at a para position of azobenzene are groups which do not take on a resonance structure with the benzene ring.

This is because a substituent, e.g., a carboxyl group, an amino group, or a nitro group, at a para position and an amide bond at a para position take on a resonance structure at the para position and, thereby, isomerization between a cis form (nonplanar structure) and a trans form (planar structure) of azobenzene tends to be effected thermally. Preferably, a substituent at a meta position is a group other than the nitro group. Examples of X can include organic groups represented by the following Formulae (IV), (V), or (VI).

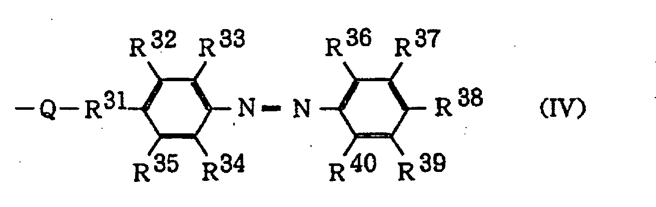

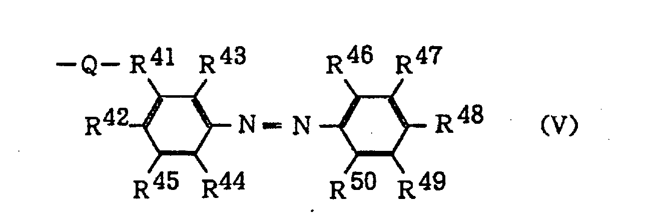

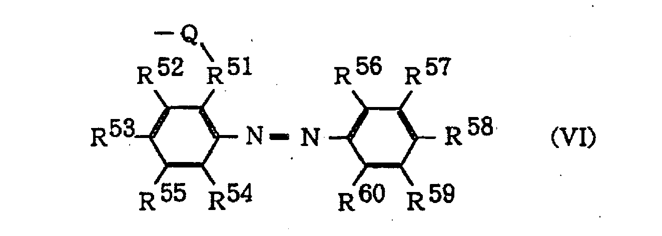

In the above-described Formulae (IV), (V), and (VI), R³¹, R⁴¹, and R⁵¹ represent independently a direct bond; an unsubstituted or a halogen atom-, hydroxyl-, amino-, nitro-, or carboxyl-substituted alkylene group having the carbon number of 1 to 20, preferably of 1 to 10, and further preferably of 1 to 4; or an unsubstituted or a halogen atom-, hydroxyl-, amino-, nitro-, or carboxyl-substituted alkenylene group having the carbon number of 2 to 20, preferably of 2 to 10, and further preferably of 2 to 4. Q represents a direct bond, an oxygen atom, a -(CH₂)ₙ-NH-CO- group, or a -(CH₂)ₙ-CO-NH- group, where n = 1 to 5. R³² to R³⁷, R³⁹, R⁴⁰, R⁴² to R⁴⁷, R⁴⁹, R⁵⁰, R⁵² to R⁵⁷, R⁵⁹, and R⁶⁰ represent independently an unsubstituted or a halogen atom-, hydroxyl-, amino-, nitro-, or carboxyl-substituted alkyl group or alkoxy group having the carbon number of 1 to 20, preferably of 1 to 10, and further preferably of 1 to 4; an unsubstituted or a halogen atom-, hydroxyl-, amino-, nitro-, or carboxyl-substituted alkenyl group or alkynyl group having the carbon number of 2 to 20, preferably of 2 to 10, and further preferably of 2 to 4; a hydroxyl group; a halogen atom; an amino group; a nitro group; or a carboxyl group. R³⁸, R⁴⁸, and R⁵⁸ represent independently an unsubstituted or a halogen atom-, hydroxyl-, amino-, nitro-, or carboxyl-substituted alkyl group or alkoxy group having the carbon number of 1 to 20, preferably of 1 to 10, and further preferably of 1 to 4; an unsubstituted or a halogen atom-, hydroxyl-, amino-, nitro-, or carboxyl-substituted alkenyl group or alkynyl group having the carbon number of 2 to 20, preferably of 2 to 10, and further preferably of 2 to 4; a hydroxyl group; or a halogen atom. Preferably, in Formula (IV), -Q-R³¹- is an intercalating group which does not take on a resonance structure with azobenzene.

Every light with a wavelength within the range from the ultraviolet region to the infrared region can be used as the light applied to effect the structural isomerization of the above-described azobenzene group, as long as the light can isomerize the azobenzene group. However, 300 nm or more is preferable because the DNA is not damaged. For example, the structural isomerization from one isomer to the other isomer can be effected by applying the light (UV light) of 300 to 400 nm, and a reverse change can be effected by applying the light (visible light) of 400 nm or more.

### EXAMPLES

The present invention will be described below in further detail with reference to examples. However the present invention is not limited to these examples.

### Synthesis example 1

### "Synthesis of DNA enzyme including azobenzene derivative"

The synthesis was conducted on the basis of the following scheme.

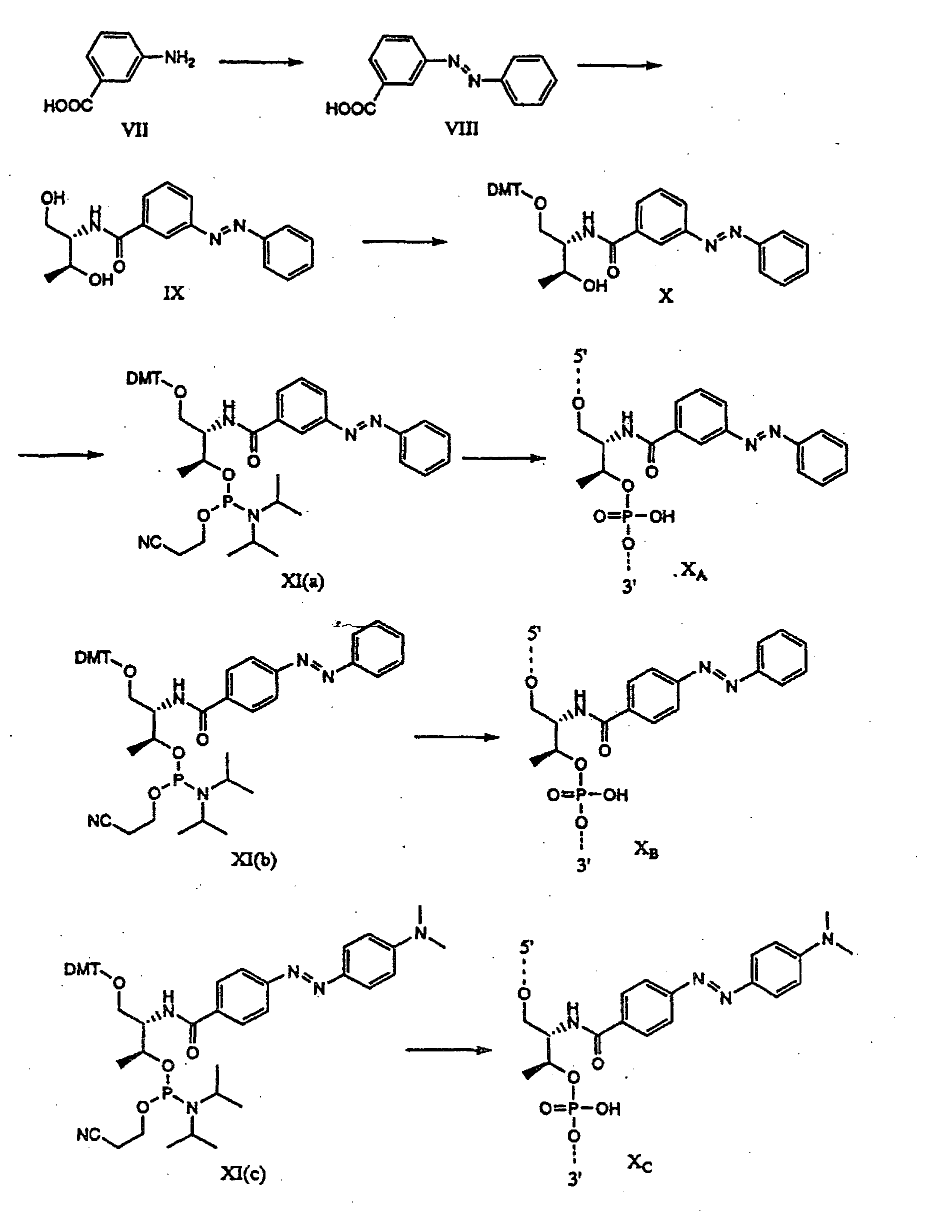

An unrefined product of 3-phenylazobenzoic acid VIII was produced by dissolving 3-aminobenzoic acid VII into acetic acid, mixing an acetic acid solution of nitrosobenzene therewith, and agitating for 12 hours at room temperature. The resulting unrefined product was refined through recrystallization by using ethanol. The resulting 3-phenylazobenzoic acid VIII and D-threoninol were reacted in N,N-dimethylformamide (DMF) in the presence of dicyclohexylcarbodiimide and 1-hydroxybenzotriazol, so that an unrefined product of Compound IX in which 3-phenylazobenzoic acid VIII and D-threoninol were bonded by an amide bond.

The resulting Compound IX was separated and refined by column chromatography and, thereafter, was reacted with 4,4'-dimethoxytrityl chloride in a pyridine-dichloromethane mixed solvent in the presence of 4-dimethylaminopyridine on the basis of the technique described in Angewandte Chemie International edition 40. 2671-2673 (2001), so that an unrefined product of Compound X, in which one hydroxyl group is protected by the 4,4'-dimethoxytrityl (DMT) group, was produced. The resulting Compound X was separated and refined by the column chromatography. Subsequently, the resulting Compound X and 2-cyanoethyl-N,N,N',N'-tetraisopropylphosphorodiamidite were reacted in acetonitrile in the presence of 1H-tetrazole on the basis of the technique described in The Journal of Organic Chemistry 62. 846-852 (1997) and Tetrahedron Letters 39. 9019-9022 (1998), so that an unrefined product of a phosphoamidite monomer XI(a), in which phosphoroamidide was added to the other hydroxyl group, was produced and, thereafter was separated and refined by the column chromatography.

A phosphoroamidite monomer XI(b) was synthesized in the same manner as that described above except that 4-phenylazobenzoic acid was used in place of 3-phenylazobenzoic acid VIII. Furthermore, a phosphoroamidite monomer XI(c) was synthesized in the same manner as that described above except that para-methyl red was used in place of 3-phenylazobenzoic acid VIII.

Finally, a chemically modified DNA enzyme including an azobenzene derivative, according to the present invention, was synthesized. In the present example, a 10-23 DNA enzyme was synthesized. For the synthesis of the chemically modified DNA enzyme, ABI394 type DNA synthesizer was used, phosphoamidite monomers XI(a) to XI(c) produced as described above and a commercially available phosphoamidite monomer corresponding to four natural bases were used, and DNA enzymes (DNA-1A: Sequence No. 4, DNA-1B: Sequence No. 5, and DNA-1C: Sequence No. 6) of the present invention having the following base sequences were synthesized. After unrefined products were produced on the basis of an usual protocol, the resulting unrefined products were refined by conducting gel refinement and high performance liquid chromatography refinement. For a comparative example, a DNA enzyme (DNA-N: Sequence No. 3) simply composed of four natural bases was synthesized in a manner similar to that described above. Each base sequence is shown in the following Table 1. In the base sequences, the underlined base sequences represent catalytically active loops.

**[Table 1]**

| DNA enzyme | Base sequence |
|---|---|
| DNA-N (Sequence No. 3) | 5' - CTGAAGGGGGCTAGCTACAACGATTCTTCCT - 3' |
| DNA-1A (Sequence No. 4) | 5' - CTGAAGGGGGCTAGCTACAACGAX_{A}TTCTTCCT - 3' |
| DNA-1B (Sequence No. 5) | 5' - CTGAAGGGGGCTAGCTACAACGAX_{B}TTCTTCCT - 3' |
| DNA-1C (Sequence No. 6) | 5' - CTGAAGGGGGCTAGCTACAACGAX_{C}TTCTTCCT - 3' |

Every DNA enzyme was identified on the basis of MALDI-TOFMS. The sequence of the RNA used as the substrate was as described below. In order to provide the substrate RNA with a fluorescence label, fluorescein isothiocyanate (FITC) represented by the following Formula: 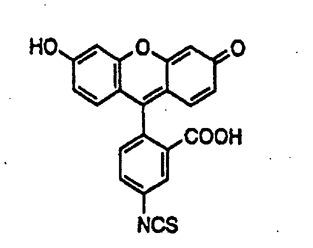 was introduced at a 5' end.

5'-(FITC)-AGGAAGAAGCCCUUCAG-3' (Sequence No. 7)

### Examples 1 to 3, Comparative example 1

### [RNA cleavage experiment]

The DNA enzymes (DNA-N: Sequence No. 3, DNA-1A: Sequence No. 4, DNA-1B: Sequence No. 5, and DNA-1C: Sequence No. 6) synthesized in Synthesis example 1 were used. The RNA cleavage experiment was conducted in accordance with the following procedure. First, 4 µL of DNA enzyme aqueous solution, 4 µL of substrate RNA aqueous solution, and furthermore, 4 µL of buffer aqueous solution were taken into a microtube, and agitation and mixing were conducted adequately at room temperature. The final concentration of each substance contained in the reaction solution was adjusted as described below.
DNA enzyme: 16 µmol/L
substrate RNA: 1.6 µmol/L
Tris-HCl: 50 mmol/L
magnesium chloride: 10 mmol/L
sodium chloride: 1 mol/L

Next, the resulting reaction solution was transferred to a constant temperature bath adjusted at 37°C, and reaction was conducted for 1 hour with respect to Comparative example 1 (DNA-N: Sequence No. 3) and Examples 1 and 2 (DNA-1A: Sequence No. 4 and DNA-1B: Sequence No. 5), and for 40 minutes with respect to Example 3 (DNA-1C: Sequence No. 6). Thereafter, 12 µL of aqueous solution containing 10 mol/L of urea and 50 mmol/L of ethylenediaminetetraacetic acid was added to terminate the reaction, and cleavage pieces of the RNA and uncleaved RNA were separated by acrylamide gel electrophoresis. Finally, FITC in the resulting gel was excited by the light of 470 nm and the fluorescence intensity at 520 nm was monitored with a fluoroimager (FLA-3000: produced by Fuji Photo Film Co., Ltd.), so that the amount of cleavage of the RNA was quantified. The cleavage results are shown in the following Table 2.

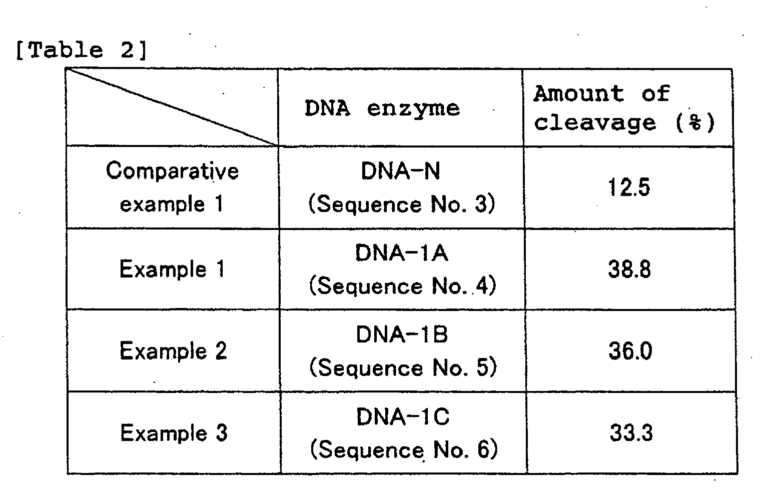

From the results shown in Table 2, it was ascertained that when a molecule having high planarity is chemically introduced at the 3'-side end of the catalytically active loop of the DNA enzyme, an RNA cleavage activity higher than that of the known DNA enzyme simply composed of natural bases is provided. Furthermore, since all three substances, meta-azo (Example 1), para-azo (Example 2), and methyl red (Example 3), have the same level of RNA cleavage activity, it is believed that if a substance is planar, intercalation and stabilization are possible.

### Examples 4 to 7, Comparative example 2

### "Optical control of RNA cleavage activity"

DNA enzymes were additionally synthesized in accordance with the method in Synthesis example 1. The base sequencies thereof are shown in the following Table 3. In the base sequences, the underlined base sequences represent catalytically active loops.

**[Table 3]**

| DNA enzyme | Base sequence |
|---|---|
| DNA-2A (Sequence No.8) | 5' - CTGAAGGGGGCTAGCTACAACGATX_{A}TCTTCCT - 3' |
| DNA-3A (Sequence No.9) | 5' - CTGAAGGGGGCTAGCTACAACGATTCX_{A}TTCC'T - 3' |

The resulting DNA enzyme was used, and a reaction solution was prepared at room temperature in the same manner as that in Examples 1 to 3. The reaction solution was transferred to a constant temperature bath at 37°C, and the reaction was conducted for a predetermined time while ultraviolet light was applied through a UV-D36C filter (produced by Asahi Techno Glass Corporation) by using a UV-A fluorescent lamp (FL6BL-A: produced by TOSHIBA CORPORATION). The intensity of the UV light under this condition was 100 µJ/cm² or less. In addition, a reaction solution having the same composition was reacted under the same condition except that no UV light was applied. Thereafter, as in Examples 1 to 3, an urea-EDTA solution was added to terminate the reaction, and cleavage pieces of the RNA and uncleaved RNA were separated by the acrylamide gel electrophoresis. Finally, a fluoroimager (FLA-3000: produced by Fuji Photo Film Co., Ltd.) was used, FITC in the resulting gel was excited by the light of 470 nm, and the fluorescence intensity at 520 nm was monitored, so that the amount of cleavage of the RNA was quantified. The cleavage results are shown in the following Table 4.

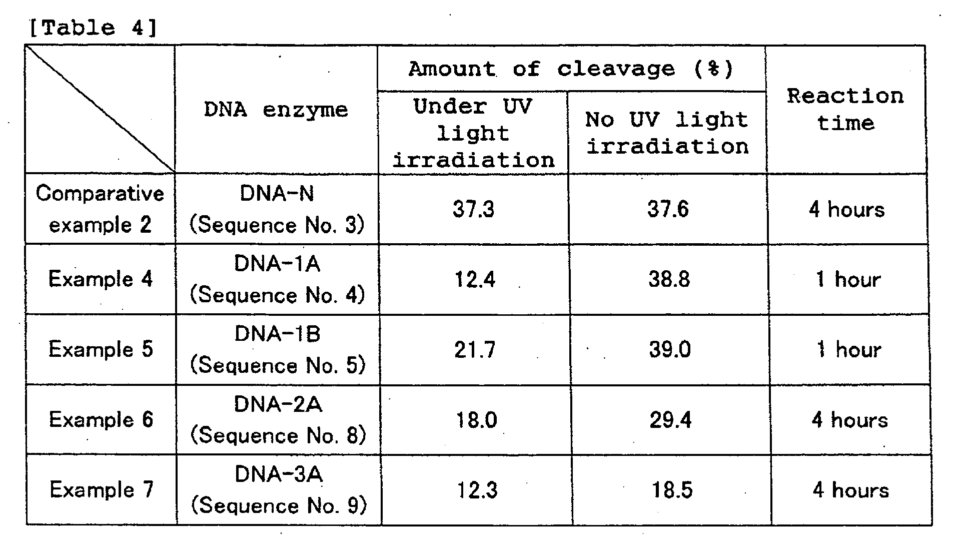

From the results shown in Table 4, it was ascertained that when the light is applied at specific wavelengths to the DNA enzyme including a residue, to which an organic group is bonded, introduced at the 3'-side end of the catalytically active loop or in oligonucleotide complementary to the substrate RNA, wherein structural isomerization between a planar structure and a nonplanar structure of the organic group is effected by the light irradiation at the specific wavelengths, the reversible structural isomerization between a planar structure and a nonplanar structure of the organic group is effected by the light irradiation at the specific wavelengths and, thereby the RNA cleavage activity can be controlled. Likewise, it is believed that the RNA cleavage activity can be controlled by the light irradiation for a DNA enzyme including nucleotide residues, to which an azobenzene group is bonded, introduced at the 3'-side end of the catalytically active loop and in oligonucleotide complementary to the substrate RNA, wherein structural isomerization between a planar structure and a nonplanar structure of the azobenzene group is effected.

### Industrial Applicability

When the high activity DNA enzyme according to the present invention is used, gene expression at a messenger RNA level can be suppressed more efficiently than ever. Furthermore, since the enzyme activity of a DNA enzyme can be controlled by the light irradiation, the gene expression can be reversibly controlled. Consequently, it is expected that the usefulness thereof is exhibited in various fields of biotechnology.

## Claims

1. A DNA enzyme (deoxyribozyme) represented by any of the following Formulae: 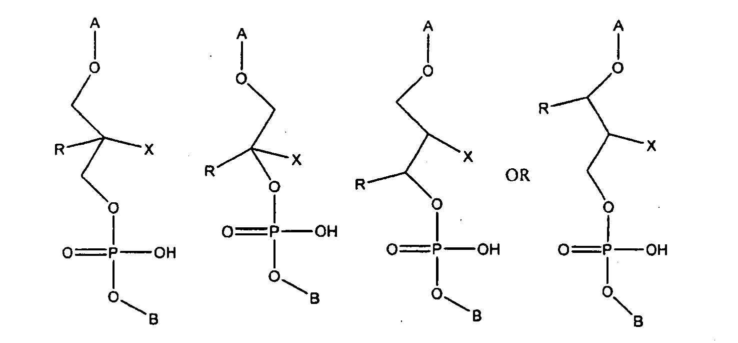 wherein A represents a catalytically active loop end, B represents a nucleotide or oligonucleotide, X represents an azobenzene group or a derivative thereof, and R represents a hydrogen atom or an alkyl group having the carbon number of 1 to 4.

2. A DNA enzyme according to claim 1, wherein X is represented by any of the following Formulae (i), (ii), or (iii): 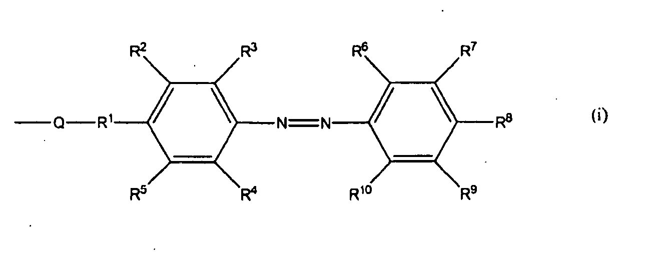 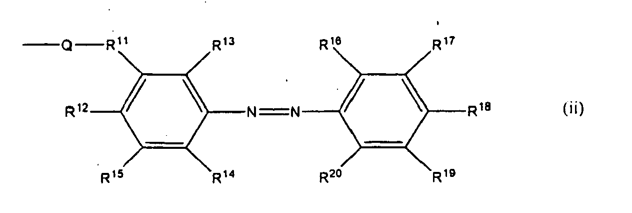 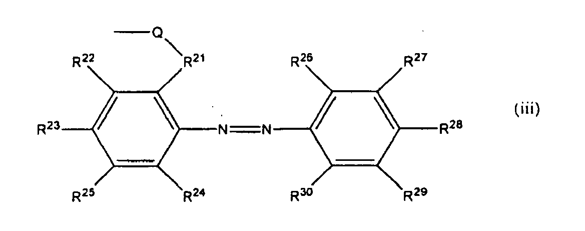 wherein R¹, R¹¹, and R²¹ represent independently a direct bond, an unsubstituted or a halogen atom-, hydroxyl-, amino-, nitro-, or carboxyl-substituted alkylene group having the carbon number of 1 to 20; or an unsubstituted or a halogen atom-, hydroxyl-, amino-, nitro-, or carboxyl-substituted alkylene group having the carbon number of 2 to 20; Q represents a direct bond, an oxygen atom, a - (CH₂)ₙ-NH-CO- group, or a - (CH₂)ₙ-CO-NH- group, wherein n= 1 to 5; and wherein R² to R¹⁰, R¹² to R²⁰, and R²² to R³⁰ represent independently an unsubstituted or a halogen atom-, hydroxyl-, amino-, nitro-, or carboxyl-substituted alkyl group or alkoxy group having the carbon number of 1 to 20, an unsubstituted or a halogen atom-, hydroxyl-, amino-, nitro-, or carboxyl-substituted alkenyl group or alkynyl group having a carbon number of 2 to 20, a hydroxyl group, a halogen atom, an amino group, a nitro group or a carboxyl group.

3. A method for controlling the activity of a DNA enzyme, **characterised by** comprising the step of applying light at specific wavelengths to the DNA enzyme including a nucleotide residue, to which an azobenzene group, or a derivative thereof, is bonded, and thereby effecting reversible structural isomerisation between a planar structure and a nonplanar structure of the azobenzene group, so as to control the RNA cleavage activity of the DNA enzyme,
wherein the DNA enzyme is represented by any of the following Formulae: 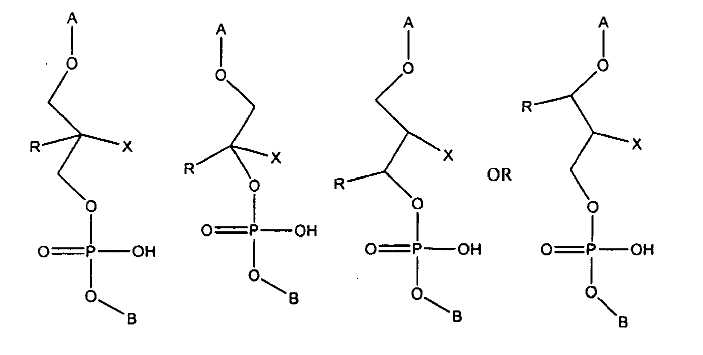 wherein A represents a catalytically active loop end; B represents nucleotide or oligonucleotide; X represents an azobenzene group or a derivative thereof; and R represents a hydrogen atom or an alkyl group having the carbon number of 1 to 4.

4. The method according to claim 3, wherein X is represented by the following Formulae (iv), (v), or (vi): 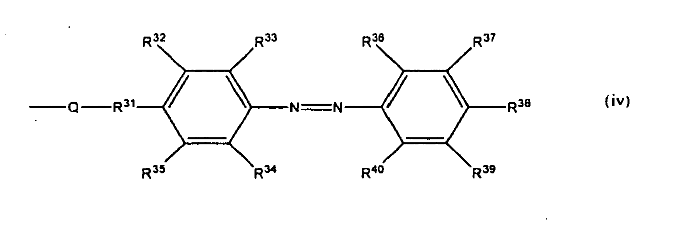 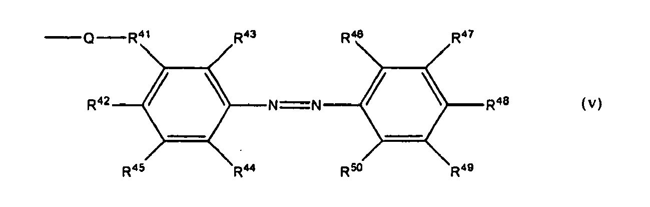 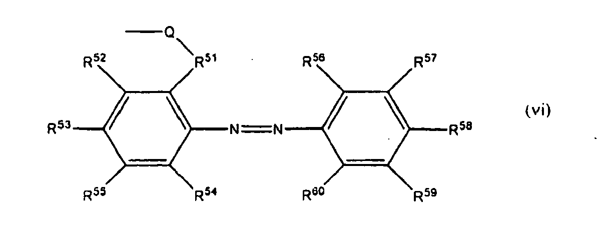 wherein R³¹, R⁴¹, and R⁵¹ represent independently a direct bond, an unsubtituted or a halogen atom-, hydroxyl-, amino-, nitro-, or carboxyl-substituted alkylene group having the carbon number of 1 to 20, or an unsubstituted or a halogen atom-, hydroxyl-, amino-, nitro-, or carboxyl-substituted alkenylene group having the carbon number of 2 to 20; Q represents a direct bond, an oxygen atom, a - (CH₂)ₙ-NH-CO- group, or a - (CH₂)ₙ-CO-NH- group, wherein n= 1 to 5; and wherein R³² to R³⁷, R³⁹, R⁴⁰, R⁴² to R⁴⁷, R⁴⁹, R⁵⁰, R⁵² to R⁵⁷, R⁵⁹, and R⁶⁰ represent independently an unsubstituted or a halogen atom-, hydroxyl-, amino-, nitro-, or carboxyl-substituted alkyl group or alkoxy group having the carbon number of 1 to 20; an unsubstituted or a halogen atom-, hydroxyl-, amino-, nitro-, or carboxyl-substituted alkenyl group or an alkynyl group having a carbon number of 2 to 20, a hydroxyl group, a halogen atom, an amino group, a nitro group or a carboxyl group, and wherein R³⁸, R⁴⁸, and R⁵⁸ represent independently an unsubstituted or a halogen atom-, hydroxyl-, amino-, nitro-, or carboxyl-substituted alkyl group or alkoxy group having the carbon number of 1 to 20, an unsubstituted or a halogen atom-, hydroxyl-, amino-, nitro-, or carboxyl-substituted alkenyl group or alkynyl group having a carbon number of 2 to 20, a hydroxyl group or a halogen atom.

## Patentansprüche

1. Ein DNA-Enzym-Deoxyribozym dargestellt durch eine der folgenden Formeln: 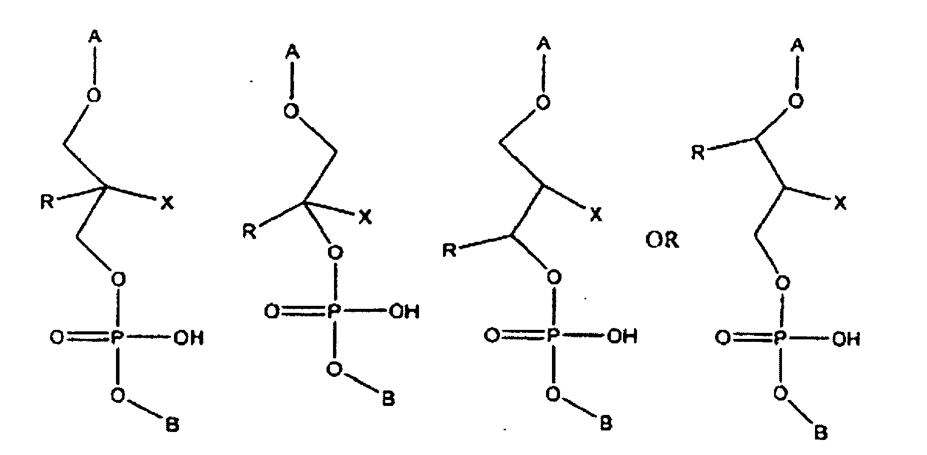 wobei A ein katalytisch aktives Schleifenende darstellt, B ein Nukleotid oder Oligonukleotid darstellt. X stellt eine Azobenzolgruppe oder ein Derivat davon dar, und R stellt ein Wasserstoffatom oder eine Alkylgruppe dar, die die Kohlenstoffzahl 1 bis 4 hat.

2. Ein DNA-Enzym gemäß Anspruch 1, wobei X durch eine der folgenden Formeln (i), (ii) oder (iii) dargestellt wird: 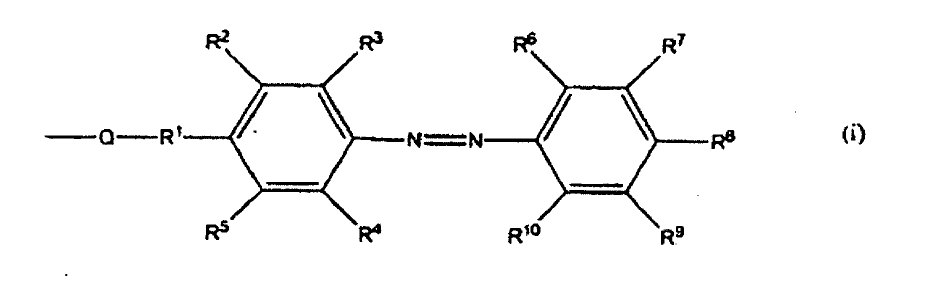 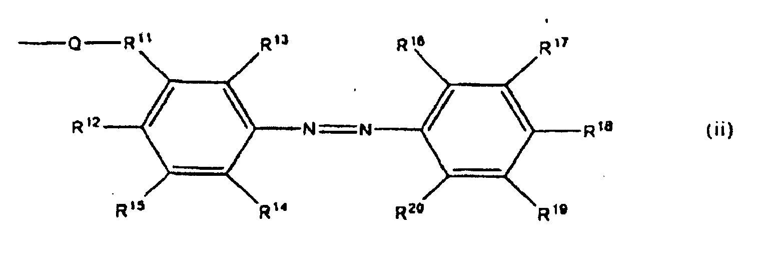 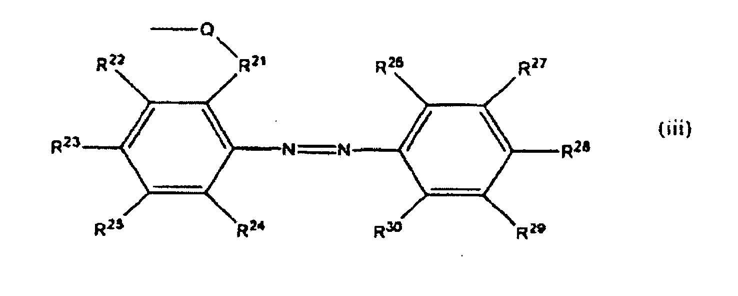 wobei R¹, R¹¹ und R²¹ unabhängig eine direkte Bindung, eine nicht substituierte oder eine Halogenatom-, Hydroxyl-, Amino-, Nitro- oder Carboxyl-substituierte Alkylengruppe, die die Kohlenstoffzahl 1 bis 20 hat, oder eine nicht substituierte oder eine Halogenatom- Hydroxyl-, Amino-, Nitro- oder Carboxyl-substituierte Alkylengruppe darstellen, die die Kohlenstoffzahl 2 bis 20 hat; Q stellt eine direkte Bindung, ein Sauerstoffatom, eine - (CH₂)ₙ-NH-CO-Gruppe oder eine - (CH₂)ₙ-CO-NH-Gruppe dar, wobei n= 1 bis 5; und wobei R² bis R¹⁰, R¹² bis R²⁰ und R²² bis R³⁰ unabhängig eine nicht substituierte oder eine Halogenatom-, Hydroxyl-, Amino-, Nitro- oder Carboxyl-substituierte Alkylgruppe oder Alkoxygruppe, die die Kohlenstoffzahl 1 bis 20 hat, eine nicht substituierte oder eine Halogenatom-, Hydroxyl-, Amino-, Nitro- oder Carboxyl-substituierte Alkenylgruppe oder Alkynylgruppe, die die Kohlenstoffzahl 2 bis 20 hat, eine Hydroxylgruppe, ein Halogenatom, eine Aminogruppe, eine Nitrogruppe oder eine Carboxylgruppe darstellen.

3. Eine Methode zur Kontrolle der Aktivität eines DNA-Enzyms, **gekennzeichnet dadurch, dass** der Schritt des Applizierens von Licht bei spezifischen Wellenlängen auf das DNA-Enzym umfasst ist, einschließlich eines Nukleotidrestes, an welchem eine Azobenzolgruppe oder ein Derivat davon gebunden ist, und dadurch eine reversible strukturelle Isomerisierung zwischen einer planaren Struktur und einer nicht-planaren Struktur der Azobenzolgruppe bewirkt, um so die RNA-Spaltungsaktivität des DNA-Enzyms zu kontrollieren.
Wobei das DNA-Enzym durch eine der folgenden Formeln dargestellt wird: 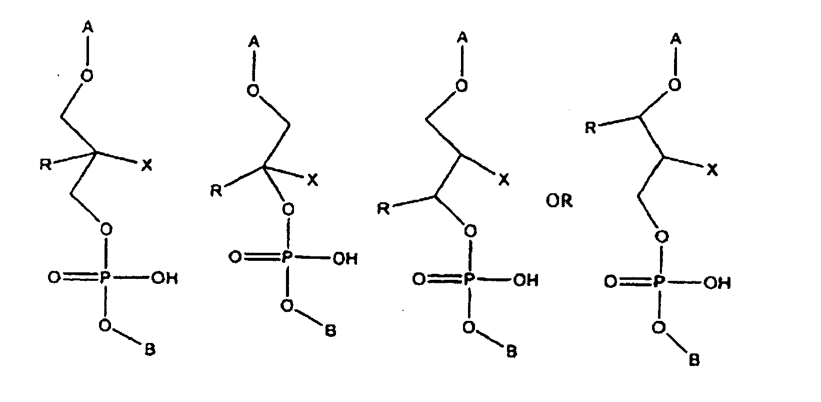 wobei A ein katalytisch aktives Schleifenende darstellt; B stellt ein Nukleotid oder Oligonukleotid dar. X stellt eine Azobenzolgruppe oder ein Derivat davon dar; und R stellt ein Wasserstoffatom oder eine Alkylgruppe dar, die die Kohlenstoffzahl 1 bis 4 hat.

4. Eine Methode gemäß Anspruch 3, wobei X durch eine der folgenden Formeln (iv), (v) oder (vi) dargestellt wird: 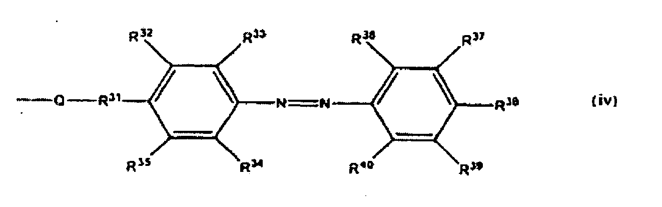 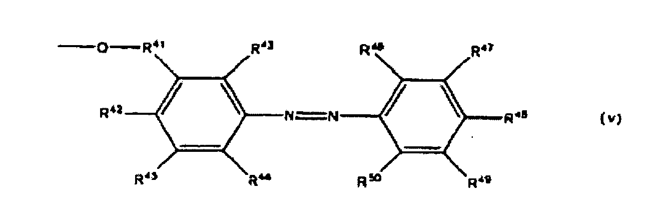 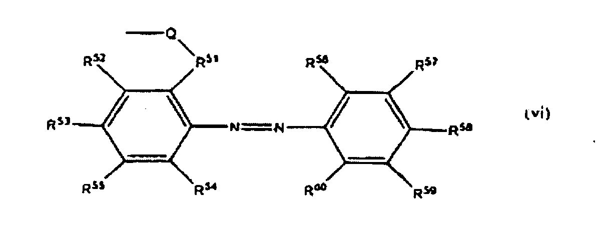 wobei R³¹, R⁴¹ und R⁵¹ unabhängig eine direkte Bindung, eine nicht substituierte oder eine Halogenatom-, Hydroxyl-, Amino-, Nitro- oder Carboxyl-substituierte Alkylengruppe, die die Kohlenstoffzahl 1 bis 20 hat, oder eine nicht substituierte oder eine Halogenatom- Hydroxyl-, Amino-, Nitro- oder Carboxyl-substituierte Alkenylengruppe darstellen, die die Kohlenstoffzahl 2 bis 20 hat; Q stellt eine direkte Bindung, ein Sauerstoffatom, eine - (CH₂)ₙ-NH-CO-Gruppe oder eine - (CH₂)ₙ-CO-NH-Gruppe dar, wobei n= 1 bis 5; und wobei R³² bis R³⁷, R³⁹, R⁴⁰, R⁴² bis R⁴⁷, R⁴⁹, R⁵⁰, R⁵² bis R⁵⁷, R⁵⁹ und R⁶⁰ unabhängig eine nicht substituierte oder eine Halogenatom-, Hydroxyl-, Amino-, Nitro- oder Carboxyl-substituierte Alkylgruppe oder Alkoxygruppe, die die Kohlenstoffzahl 1 bis 20 hat, eine nicht substituierte oder eine Halogenatom-, Hydroxyl-, Amino-, Nitro- oder Carboxyl-substituierte Alkenylgruppe oder eine Alkynylgruppe, die die Kohlenstoffzahl 2 bis 20 hat, eine Hydroxylgruppe, ein Halogenatom, eine Aminogruppe, eine Nitrogruppe oder eine Carboxylgruppe darstellen; und wobei R³⁸, R⁴⁸ und R⁵⁸ unabhängig eine nicht substituierte oder eine Halogenatom-, Hydroxyl-, Amino-, Nitro- oder Carboxyl-substituierte Alkylgruppe oder Alkoxygruppe, die die Kohlenstoffzahl 1 bis 20 hat, eine nicht substituierte oder eine Halogenatom-, Hydroxyl-, Amino-, Nitro- oder Carboxyl-substituierte Alkenylgruppe oder Alkynylgruppe, die die Kohlenstoffzahl 2 bis 20 hat, eine Hydroxylgruppe oder ein Halogenatom darstellen.

## Revendications

1. Un enzyme ADN (deoxyribozyme) représenté par une quelconque des formules suivantes : 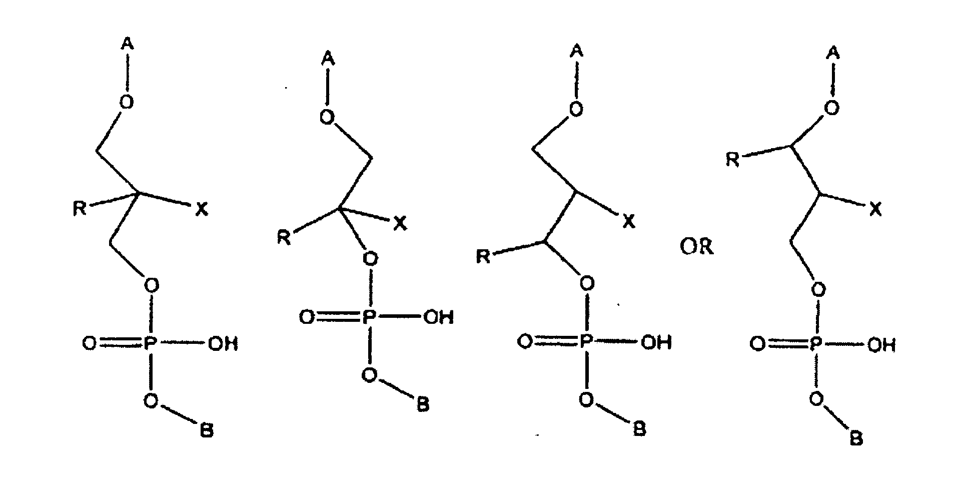 Où A représente une extrémité de boucle catalytique active, B représente un nucléotide ou oligonucleotide, X représente un groupe azobenzène ou un de ses dérivatifs, et R représente un atome d'hydrogène ou un groupe alkyle possédant le nombre carbone de 1 à 4.

2. Un enzyme ADN selon Revendication 1, où X est représenté par une quelconque des formules suivantes (i), (ii), ou (iii): 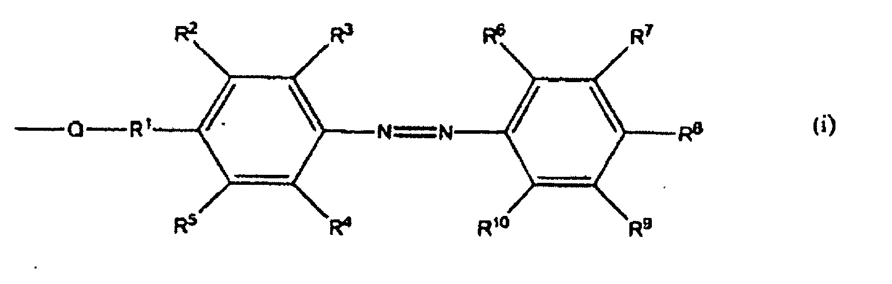 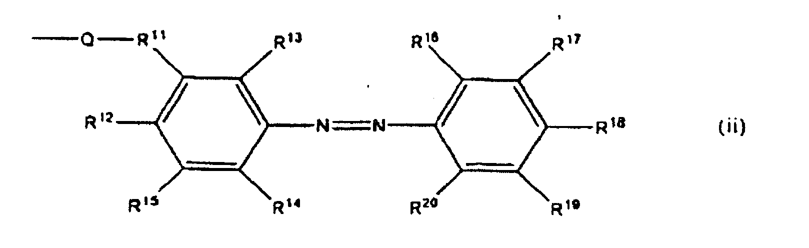 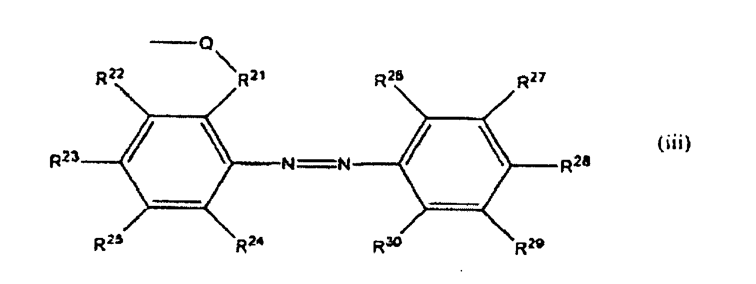 où R¹, R¹¹, et R²¹ représentent indépendamment une association directe, un groupe non substitué ou un groupe halogène substitué atome-, hydroxyle-, amino-, nitro-, ou un alkylène carboxyle-substitué possédant le nombre carbone de 1 à 20 ; ou un groupe halogène substitué atome-, hydroxyle-, amino-, nitro-, ou carboxyle alkylène possédant le nombre carbone de 2 à 20 ; Q représente une association directe, un atome d'oxygène, un groupe - (CH₂)ₙ- NH -CO-, ou un groupe (CH₂)ₙ - CO - NH-, où n= 1 à 5 ; et où R² à R¹⁰, R¹² à R²⁰, et R²² à R³⁰ représentent indépendamment un groupe non substitué ou un groupe substitué atome-, hydroxyle-, amino-, nitro-, ou carboxyle alkylène ou un groupe alkoxy possédant le nombre carbone de 1 à 20, un groupe non substitué ou un groupe halogène substitué atome-, hydroxyle-, amino-, nitro-, ou un alkylène carboxyle-substitué possédant le nombre carbone de 2 à 20, un groupe hydroxyle, un atome halogène, un groupe amino, un groupe nitro ou un groupe carboxyle.

3. Une méthode permettant de contrôler l'activité de l'enzyme ADN, **caractérisé par** l'inclusion de l'étape d'application de lumière à des longueurs d'ondes spécifiques à l'enzyme ADN, y compris un résidu de nucléotide, auquel un groupe azobenzène ou un de ses dérivatifs, est associé, et par conséquent effectue une isomérisation structurelle réversible entre la structure planaire et la structure non planaire du groupe azobenzène, afin de contrôler l'activité de fendage RNA de l'enzyme ADN, où l'enzyme est représenté par une quelconque des formules suivantes : 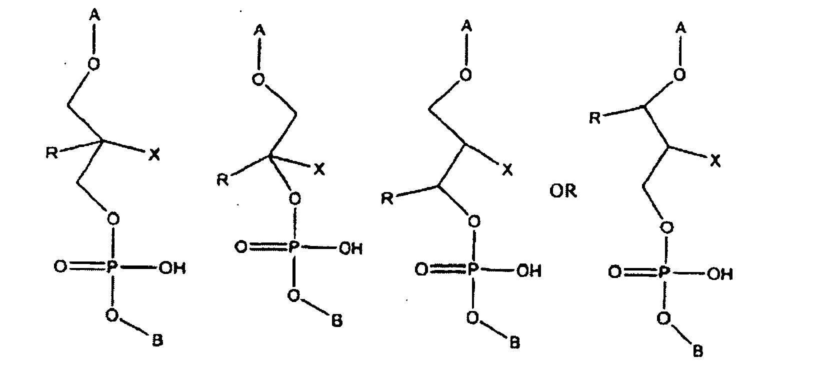 où A représente une extrémité de boucle catalytique active, B représente un nucléotide ou oligonucléotide, X représente un groupe azobenzène ou un de ses dérivatifs, et R représente un atome d'hydrogène ou un groupe alkyle possédant le nombre carbone de 1 à 4.

4. la méthode selon Revendication 3, où X est représenté par les formules suivantes (iv), (v), ou (vi) : 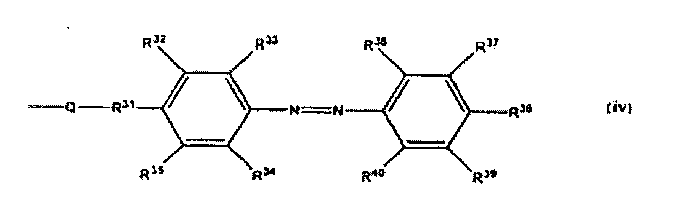 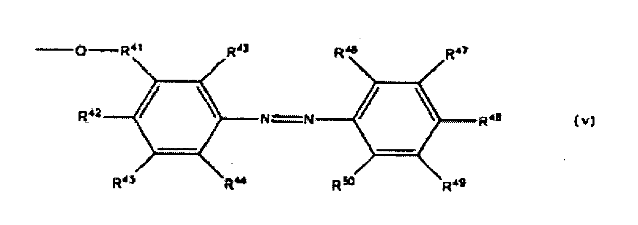 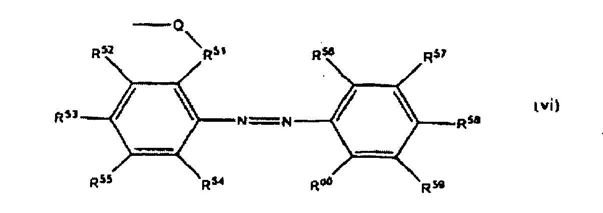 où R³¹, R⁴¹, et R⁵¹ représentent indépendamment un groupe non substitué ou un groupe halogène substitué atome-, hydroxyle-, amino-, nitro-, ou carboxyle alkylène possédant le nombre carbone de 1 à 20, ou un groupe non substitué ou un groupe halogène atome-, hydroxyle-, amino-, nitro-, ou un groupe substitué carboxyle alkenylène possédant le nombre carbone de 2 à 20 ; Q représente une association directe, un atome d'oxygène, un groupe - (CH₂)ₙ - NH -CO-, ou un groupe (CH₂)ₙ - CO - NH-, où n= 1 à 5 ; et où R³² à R³⁷, R³⁹, R⁴⁰, R⁴² à R⁴⁷, R⁴⁹, R⁵⁰, R⁵² à R⁵⁷, R⁵⁹ et R⁶⁰ représentent indépendamment un groupe non substitué ou un groupe halogène atome-, hydroxyle-, amino-, nitro-, ou carboxyle-substitué alkylène ou un groupe alcyne possédant le nombre carbone de 1 à 20, un groupe hydroxyle, un atome halogène, un groupe amino, un groupe nitro un groupe carboxyle ; et où R³⁸, R⁴⁸, et R⁵⁸ représentent indépendamment un groupe non substitué ou un groupe halogène substitué atome-, hydroxyle-, amino-, nitro-, ou carboxyle alkylène ou un groupe alkoxy possédant le nombre carbone de 1 à 20, un groupe non substitué ou un groupe halogène substitué atome-, hydroxyle-, amino-, nitro-, ou un groupe substitué alkylène carboxyle possédant le nombre carbone de 2 à 20, un groupe hydroxyle ou un atome halogène.
